(19) Europäisches Patentamt

European Patent Office

Office européen des brevets

(11) Veröffentlichungsnummer: **0 554 739 A2**

## (12) EUROPÄISCHE PATENTANMELDUNG

(21) Anmeldenummer: **93101047.4**

(22) Anmeldetag: **23.01.93**

(51) Int. Cl.5: **C07C 19/08**

(30) Priorität: **01.02.92 DE 4202906**

(43) Veröffentlichungstag der Anmeldung:
**11.08.93 Patentblatt 93/32**

(84) Benannte Vertragsstaaten:
**AT BE CH DE DK ES FR GB GR IE IT LI LU NL
PT SE**

(71) Anmelder: **Solvay Fluor und Derivate GmbH
Hans-Böckler-Allee 20
W-3000 Hannover 1(DE)**

(72) Erfinder: **Fazniewscy, Karl-Heinz
Im Gesenk 8
W-3160 Lehrte(DE)**
Erfinder: **Kaudewitz, Peter
Lessingstrasse 14
W-7107 Bad Wimpfen(DE)**
Erfinder: **Rieland, Matthias, Dr.
Süsseroder Strasse 4a
W-3000 Hannover 71(DE)**
Erfinder: **Rudolph, Werner, Dr.
Oderstrasse 38
W-3000 Hannover 71(DE)**
Erfinder: **Sprenger, Oskar
Mansfeldstrasse 57
W-7107 Bad Wimpfen(DE)**
Erfinder: **Feist, Rudi, Dr.
2 Georgetown North
Greenwich, CT 06831(US)**

(74) Vertreter: **Lauer, Dieter, Dr.
c/o Solvay Deutschland GmbH,
Hans-Böckler-Allee 20
W-3000 Hannover 1 (DE)**

(54) **Herstellung von Hexafluorethan durch pyrolytische Zersetzung von Trifluorbromomethan.**

㉗ Beschrieben wird die Herstellung von Hexafluorethan durch pyrolytische Zersetzung von Trifluormethylbromid an erhitzten Kontakten, die mit einem Platingruppenmetall beschichtet sind oder daraus bestehen. Das Verfahren gestattet die Überführung von $CF_3Br$ aus z.B. Feuerlöscheinrichtungen oder aus der Lösungsmittelrückgewinnung in verwertbare Wertstoffe.

Die Erfindung bezieht sich auf ein verfahren zur Herstellung von Hexafluorethan unter pyrolytischer Zersetzung von Trifluormethylhalogeniden in der Gasphase an erhitzten Kontakten.

Hexafluorethan ist beispielsweise als Treibmittel, als Kältemittel oder als Ätzgas verwendbar. Es kann beispielsweise durch Addition von elementarem Fluor an Tetrafluorethylen oder durch einen durch Eisen katalysierten Chlor-Fluor-Austausch an Chlorpentafluorethan mit Fluorwasserstoff hergestellt werden. Hexafluorethan entsteht auch bei der pyrolytischen Zersetzung von Trifluormethylbromid an erhitzten Quarzrohren. Bei niedrigen Temperaturen, beispielsweise bei 621 °C, stellt sich ein Gleichgewicht aus Trifluormethylbromid, Brom und Hexafluorethan jedoch erst nach 33 Tagen ein, bei 722 °C in etwa 5 Stunden. Oberhalb dieser Temperatur kommt es zu Nebenreaktionen von $CF_3$-Radikalen mit Siliziumdioxid. Oberhalb von 900 °C, wo die Gleichgewichtseinstellung besonders schnell erfolgen sollte, beobachtet man Abscheidung von Kohlenstoff und elementarem Fluor. Dieses Laborverfahren ist technisch deshalb uninteressant.

Aufgabe der vorliegenden Erfindung ist es, ein technisch durchführbares Verfahren anzugeben, mit dem möglichst selektiv Hexafluorethan durch pyrolytische Zersetzung von Trimethylhalogeniden an erhitzten Kontakten in der Gasphase hergestellt werden kann. Diese Aufgabe wird durch das in den Ansprüchen angegebene verfahren gelöst.

Die Erfindung beruht auf der Erkenntnis, daß bei Verwendung erhitzter Kontakte, die auf der mit dem Ausgangsmaterial in Kontakt stehenden Seite mit einem Platingruppenmetall beschichtet sind oder die aus einem Platingruppenmetall bestehen, die Bildung von Nebenprodukten wirksam unterdrückt wird.

Das erfindungsgemäße Verfahren zur Herstellung von Hexafluorethan ($C_2F_6$) unter pyrolytischer Zersetzung von Trifluormethylhalogeniden in der Gasphase an erhitzten Kontakten ist dadurch gekennzeichnet, daß man $CF_3Br$, gegebenenfalls im Gemisch mit $CF_3H$, einsetzt und die pyrolytische Zersetzung bei einer Temperatur zwischen 650 °C und 1000 °C in einem Reaktor durchführt, dessen erhitzte Kontakte mit einem Platingruppenmetall beschichtet sind oder daraus bestehen.

Die nicht erhitzten Reaktorteile bestehen zweckmäßig aus inertem Material, beispielsweise aus Aluminiumoxid. Die nicht erhitzten Katalysatorteile können aber ebenfalls mit einem Platingruppenmetall beschichtet sein oder daraus bestehen.

Setzt man ein Gemisch von $CF_3Br$ und $CF_3H$ ein, kann es bis 50 Gew.-% $CF_3H$ enthalten, z.B. 5 bis 30 Gew.-%.

Bevorzugtes Ausgangsmaterial ist $CF_3Br$ ohne Zusatz von $CF_3H$. Sauerstoff, Luft oder Wasserstsoff werden vorteilhaft nicht beigemischt.

Man verwendet zweckmäßig rohrförmige Reaktoren. Die erhitzten Kontakte sind zweckmäßig flächenförmig. Sie können beispielsweise als Bleche parallel zur Richtung des durchströmenden Gases angeordnet sein. Sehr gut läßt sich das erfindungsgemäße Verfahren in Rohrreaktoren durchführen, die innen mit einem Platingruppenmetall, insbesondere Platin, beschichtet sind oder aus Platin bestehen. Solche Rohrreaktoren werden durch außen liegende Heizeinrichtungen beheizt.

Besonders gut geeignet sind Ringspaltreaktoren. Solche Reaktoren sind gewöhnlich rohrförmig. Der Reaktionsraum wird durch den Ringspalt gebildet, der zwischen einem äußeren Mantelrohr und einem gewöhnlich konzentrisch dazu angeordneten innereren Rohr (das auch massiv sein kann) gebildet wird. Man kann das äußere Mantelrohr und/oder das Innenrohr beheizen. die jeweils beheizte Fläche bzw. die jeweils beheizten Flächen weisen auf der Reaktionsraumseite eine Beschichtung aus einem Platingruppenmetall, vorzugsweise Platin, auf.

Es hat sich herausgestellt, daß eine kurze Verweilzeit für eine weitgehende Unterdrückung von Nebenreaktionen förderlich ist. Die Verweilzeit liegt vorzugsweise im Bereich von 0,1 bis 10 sec, insbesondere 0,5 bis 2 sec. Eine hohe Temperatur beschleunigt die Einstellung der Gleichgewichtsreaktion. Vorzugsweise arbeitet man im Bereich von 700 bis 950 °C, insbesondere 800 bis 950 °C.

Man kann bei Umgebungsdruck arbeiten oder auch bei höheren Drücken, beispielsweise im Bereich von 1 bis 5 bar.

Das erfindungsgemäße Verfahren läßt sich besonders gut im Rahmen der Wiederverwertung von $CF_3Br$ aus der Kältemittel-, Lösemittel-, Treibgas- und Feuerlöschmittel-Aufarbeitung einsetzen. Aus $CF_3Br$ kann auf diese Weise ein wertvolles Wertprodukt hergestellt werden.

Die folgenden Beispiele sollen das erfindungsgemäße Verfahren weiter erläutern, ohne es in seinem Umfang einzuschränken.

Beispiel 1:

Herstellung von Hexafluorethan in einem Ringspaltreaktor.

1.1 Verwendeter Reaktor

Verwendet wurde ein Ringspaltreaktor, dessen äußerer Mantel aus Aluminiumoxid besteht. Er weist ein inneres, konzentrisch im äußeren Mantelrohr angeordnetes Rohr auf, das innen elektrisch beheizt werden kann und innen eine Temperaturmeßeinrichtung aufweist. Der sich über eine Länge von 1,3 m erstreckende, durch den Zwischenraum zwischen den Rohren gebildete Ringspalt stellt den Reaktionsraum dar. Sein Volumen beträgt etwa 3 1. Das innere, beheizbare Rohr ist auf der den Reaktionsraum begrenzenden Seite, die die beheizbare Kontaktfläche darstellt, mit Platin beschichtet. Der Reaktor weist keine, mit dem Reaktionsgemisch in Berührung kommenden Teile aus Nickel oder Siliziumdioxid auf.

1.2 Durchführung

Das Innenrohr des Reaktors wurde erhitzt (Heiztemperatur: 950 °C). $CF_3Br$ wurde unter Sauerstoffausschluß verdampft und vorgewärmt. Es strömte dann gasförmig durch den Ringspalt und passierte dabei die heiße Kontaktfläche aus Platin. Nach dem Verlassen des Ringspaltes wurde die heiße Reaktionsmischung zur Vorwärmung des $CF_3Br$-Frischgases benutzt und dabei selbst abgekühlt. Entstandenes Brom wurde durch Absorption in KOH-Lauge von den organischen Bestandteilen abgetrennt und das verbleibende Gas hinsichtlich der organischen Bestandteile analysiert.

Beispiele 2 bis 9:

Das Beispiel 1 wurde unter Variation von Heiztemperatur und Verweilzeit (variiert durch unterschiedliche Mengenströme) wiederholt. In den Beispielen 4, 8 und 9 wurde ein Gemisch aus $CF_3Br$ und $CF_3H$ eingesetzt.

Die Daten (Heiztemperatur, Durchsätze, Verweilzeiten und analytische Daten des gebildeten Produktgases) der Beispiele 1 bis 9 sind in Tabelle 1 zusammengestellt.

Tabelle 1: Verfahrensparameter und Produktanalyse der Beispiele 1 bis 9

| Bei-spiel | Heiz-temp. [°C] | Durchsatz $CF_3H$ [g/min] | Durchsatz $CF_3Br$ [g/min] | Verweil-zeit [sec] | Gebilde-tes $CF_4$ [Gew.-%] | Gebilde-tes $C_2F_6$ [Gew.-%] | $CF_3Br$ [Gew.-%] | $CF_3H$ [Gew.-%] |
|---|---|---|---|---|---|---|---|---|
| 1 | 950.00 | 0.00 | 150.00 | 5.96 | 0.54 | 27.83 | 67.44 | 0.00 |
| 2 | 950.00 | 0.00 | 300.00 | 2.98 | 0.33 | 23.51 | 74.80 | 0.00 |
| 3 | 950.00 | 0.00 | 600.00 | 1.49 | 0.21 | 18.02 | 78.84 | 0.00 |
| 4 | 950.00 | 70.00 | 150.00 | 2.99 | 0.19 | 17.59 | 61.90 | 4.58 |
| 5 | 860.00 | 0.00 | 150.00 | 5.96 | 0.30 | 27.70 | 69.72 | 0.07 |
| 6 | 860.00 | 0.00 | 300.00 | 2.98 | 0.13 | 18.84 | 78.47 | 0.00 |
| 7 | 825.00 | 0.00 | 300.00 | 2.98 | 0.01 | 3.97 | 94.06 | 0.55 |
| 8 | 825.00 | 75.00 | 300.00 | 1.94 | 0.01 | 9.56 | 63.61 | 18.28 |
| 9 | 825.00 | 150.00 | 300.00 | 1.44 | 0.01 | 8.74 | 56.80 | 25.26 |

Der Tabelle 1 kann entnommen werden, daß bei erhöhter Temperatur ein erhöhter Durchsatz mit ähnlichen Ergebnissen möglich ist. Bei geringer Verweilzeit ist das Verhältnis zwischen gebildetem Hexafluorethan und Tetrafluormethan besonders hoch. Zusatz von $CF_3H$ erhöht den Umsatz, allerdings entsteht hier $C_2F_5Br$ als Nebenprodukt.

4

$C_2F_6$ kann durch Destillation bei niedriger Temperatur und/oder erhöhtem Druck aus der Reaktionsmischung isoliert werden.

**Patentansprüche**

1. Verfahren zur Herstellung von Hexafluorethan ($C_2F_6$) unter pyrolytischer Zersetzung von Trifluormethylhalogeniden in der Gasphase an erhitzten Kontakten, dadurch gekennzeichnet, daß man $CF_3Br$, gegebenenfalls im Gemisch mit $CF_3H$, einsetzt und die pyrolytische Zersetzung bei einer Temperatur zwischen 650 °C und 1000 °C in einem Reaktor durchführt, dessen erhitzte Kontakte mit einem Platingruppenmetall beschichtet sind oder daraus bestehen.

2. Verfahren nach Anspruch 1, dadurch gekennzeichnet, daß man $CF_3Br$ als Ausgangsmaterial einsetzt.

3. Verfahren nach Anspruch 1 oder 2, dadurch gekennzeichnet, daß die Kontakte flächige Formen aufweisen.

4. Verfahren nach einem der vorhergehenden Ansprüche, dadurch gekennzeichnet, daß die Kontakte durch die mit Platin beschichteten Innenflächen eines Rohrreaktors gebildet werden.

5. Verfahren nach einem der Ansprüche 1 bis 3, dadurch gekennzeichnet, daß man die pyrolytische Zersetzung in einem Ringspaltreaktor durchführt.

6. Verfahren nach einem der vorhergehenden Ansprüche, dadurch gekennzeichnet, daß die Verweilzeit im Bereich von 0,1 sec bis 10 sec liegt.

7. Verfahren nach Anspruch 6, dadurch gekennzeichnet, daß der Druck im Bereich von Umgebungsdruck bis 5 bar liegt.

8. Verfahren nach Anspruch 1, dadurch gekennzeichnet, daß die Temperatur im Bereich von 700 °C bis 950 °C liegt.

9. Verfahren zur Überführung von $CF_3Br$ in Wertstoffe im Rahmen der Wertstoff-Rückgewinnung, dadurch gekennzeichnet, daß man ein Verfahren nach einem der Ansprüche 1 bis 8 anwendet.